# EUROPEAN PATENT SPECIFICATION

(11) **EP 0 619 096 B1**
(45) Date of publication and mention of the grant of the patent: **24.11.1999**
(21) Application number: 94830079.3
(22) Date of filing: 22.02.1994
(51) Int. Cl.: A61B 5/14

(54) **Needle assembly for a blood sample collection**
Blutprobennadelanordnung
Aiquille d'échantillon de sang

(30) Priority: 22.02.1993 JP 7274393; 17.12.1993 JP 31825393
(43) Date of publication of application: 12.10.1994
(73) Proprietor: Suzuki, Issei, Yamato-shi Kanagawa-ken (JP)
(72) Inventor: Suzuki, Issei, Kanagawa-ken, Giappone (JP); Misawa, Hiroyasu, c/o Misawa Medical Ind.Co.,Ltd., Edogawa-ku, Tokyo (JP); Misawa, Kosei, c/o Misawa Medical Ind.Co.,Ltd., Edogawa-ku, Tokyo (JP)
(74) Representative: Mannucci, Gianfranco, Dott.-Ing.

(56) References cited:
- EP-A- 0 077 441
- EP-A- 0 146 691
- EP-A- 0 180 702
- EP-A- 0 478 459
- DE-A- 3 229 783
- US-A- 4 207 870
- US-A- 4 416 290

## Description

### BACKGROUND OF THE INVENTION

The present invention relates to a needle assembly for evacuated blood collection tubes or containers, one end of which needle is inserted into the vein of a blood examinee, and the other end is communicated with the interior of a vacuum, blood collecting container so as to be able to collect blood in an amount responsive to the negative pressure in said container.

A needle assembly for evacuated blood collection tubes or containers of this kind is known from DE-C-3229783. This assembly is illustrated in FIG. 1 of the attached drawings. Therein a needle cannula support A is secured to the middle portion of a stainless needle cannula B, and one end of the needle cannula support A is provided with a rubber cap C for covering the one end of the needle cannula B. With such kind of needle assembly, the other end i.e. the tip of the needle cannula B is inserted into the vein of a blood examinee in collecting blood, and a blood collection container is fitted from one end of the needle cannula B while covering the one end with the rubber cap C and communicating with the interior of the blood collection container whereafter it can be confirmed for the first time whether the needle has accurately entered the vein of the blood examinee. This needs a plurality of insertions to give a discomfort to the blood examinee or to release the negative pressure pre-set to the blood collection container so that it becomes unable to collect a desired amount of blood or it becomes necessary to use other blood collection tubes.

To remove such demerits, a variety of needle assemblies have been proposed that are constructed in such a way that when inserted into the vein of the blood examinee a flow-out of blood can be confirmed. One known example is such that a needle cannula is divided into two sections, one of which is to be inserted into the vein and the other to be inserted into a vacuum blood collection container, a member of connecting these two needle sections is provided with a transparent blood flow passage, and it is confirmed through this passage whether the blood is flowing or not. Alternative known example is such that the middle portion of a needle cannula is bored with a hole which is circumferentially formed with a blood flow confirmation chamber.

To refer to a needle assembly where the needle cannula is constituted by two needle sections, of the known needle assemblies for evacuated blood collection tubes so far proposed, the member of connecting the two needle sections requires a lot of components whereby manufacturing steps and cost increase. Moreover, unless the air not only inside the needle but also in the transparent blood flow passage within the member of connecting the two needle sections flows out, the blood does not flow, so that the structure to settle these problems becomes complicated.

On the other hand, according to a needle assembly where the needle cannula is bored with a hole which is circumferentially formed with a blood flow confirmation chamber, the confirmation chamber must be retained under negative pressure or released to the outside, and therefore as in the above case, the structure becomes complicated and the manufacturing cost increases.

Furthermore, such kinds of blood collection needle assemblies are used for collecting multiple samples of blood from a blood examinee, ordinarily changing the blood collection container, and each time a sample of blood is collected the rubber cap at one end of the needle cannula results in repeated boring. Generally, the tip of a needle is obliquely cut while the tip point is sharpened (lancet-shaped). However, since the tip point is positioned in the outer periphery of a needle cannula it is feared that in manufacturing needle assemblies for blood sampling the rubber cap might be impaired when it is mounted to the needle tip. Conventionally, therefore, needles are used that the sharpened tip point is bent toward the center axis of the needle cannula or obliquely cut while retaining the point in round shape. The needles having such shapes in tip substantially decrease the danger of impairing the rubber cap when the cap is mounted in manufacturing the needle assemblies. On the other hand, however, in the case of the former the rubber cap is apt to be hooked to the bent tip point when the cap is returned to the original position (position of covering the needle tip) when a vacuum, blood collection container is disengaged after insertion with needle, and such a needle whose tip point is bent is added with a bending step so as to increase the manufacturing cost, whereas in the case of the latter, the cutting opening of the rubber cap becomes wider when inserted with needle so that while being repeatedly replaced by other vacuum blood collection tubes the rubber cap loses its sealing property whereby such a rubber cap is not suited for repeated sampling of blood.

Furthermore, such kinds of needle assemblies are disposable so that it is desired to provide them as at low cost as possible. It is further required to gurantee safe and certain functions so that the user such as doctor or nurse may not directly contact the blood when the blood is collected.

A needle cannula according to the preamble of claim 1 is disclosed in EP-A-0478459.

### SUMMARY OF THE INVENTION

The object of the present invention is therefore to provide a needle assembly for evacuated blood collection tubes or containers, which is easy and safe for use while solving the forementioned problems, is of simple structure and can be manufactured at low cost.

In order to achieve the above object the present invention starts from a needle assembly for evacuated blood collection tubes or containers according to the preamble of claim 1, including a needle cannula one end of which is adapted for insertion into the vein of a blood examinee and the other end into a vacuum, blood collection container, a needle cannula support mounted at the middle portion of said needle cannula, and an elastomeric resilient cap mounted at the other end of said needle cannula the blood collection container is mounted liquid-tight to the needle cannula support from the other end side of the needle cannula, which side is fixed with the cap, and while piercing the elastomeric resilient cap the other end of the needle cannula is communicated with the interior of the blood collection container.

The above mentioned objects are obtained with the combinations of features of claim 1. Additional advantageous features are set forth in the dependent claims.

Preferably, the longitudinal path in the needle cannula support may be circularly formed around the needle cannula.

Alternatively, the longitudinal path in the needle cannula support may be oval-shaped in section, and it may be constructed to be brought into partial contact with the outer periphery of the needle cannula or it may consist of a plurality of paths extending along the longitudinal direction of the outer periphery of the needle cannula.

Further, preferably, one end of the radial path in the needle cannula support may communicate with the inward end of said longitudinal path and the other end may open to the outside to allow this open end to be anchored with the air-permeable, liquid liquid repellant or impermeable plug.

The air-permeable, blood repellant plug may be comprised preferably of a paper mass. Instead, it may be constituted by a synthetic resinous mass.

Furthermore, the inner edge of the open end of said radial path may be formed spreading outwardly in tapered shape.

Furthermore, preferably, the end of the needle cannula, which is covered with an elastomeric resilient cap, may extend in parallel with the axis of the needle cannula and may be provided with a pointed tip positioning inner than the outer circumference of the needle cannula.

In the needle assembly for evacuated blood collection tubes according to the invention, which is constructed as aforementioned, the needle cannula support made of a translucent or transparent material is provided at the middle portion of a single needle cannula, the blood flow confirmation passage is formed within the needle cannula support, said passage communicating with the tip point at the end side of the needle cannula, said end side being covered with the elastomeric resilient cap, the open end of said passaage is provided with an air-permeable, liquid repellant plug, and therefore it is possible to easily confirm whether the needle has accurately entered the vein of a blood examinee when blood is collected. Addtionally, by inserting a vacuum blood collection container (substantially tube) the needle cannula itself is blocked from communication with the blood flow confirmation passage within the needle cannula support whereby it is possible to collect a desired amount of blood through the needle cannula.

Alternatively, by employing a paper mass as the air-permeable, liquid repellant plug it becomes capable of constructing a needle assembly without substantially increasing the manufacturing cost. Moreover, since the liquid repellant plug is made of a paper mass the needle assembly can be easily dealt with after use, and in addition to its operational safety it will also be advantageous from the viewpoint of preventing environmental contamination when the needle assembly is disposed of.

Furthermore, because of the shape of the needle where the tip of the end of said needle cannula, which is covered with the elastomeric resilient cap, extends in parallel with the axis of the needle cannula and is pointed while positioning inner than the outer circumference of the needle cannula, the elastomeric resilient cap is no longer impaired when mounted in the assembling process of the needles. Additionally, even if vacuum, blood collection containers were repeatedly engaged and disengaged, the cutting opening neither broadens nor is hooked whereby the sealability of the cap can be sufficiently retained and even the operability can be improved.

### BRIEF DESCRIPTION OF THE DRAWINGS

The invention will now be described more in detail, by way of embodiments, with reference to the accompanying drawings in which
FIG. 1 is a schematic diagram illustrating one example of a known needle assembly;
FIG. 2 is a schematical front view of the needle assembly for evacuated blood collection tubes according to one embodiment of the present invention;
FIG. 3 is a longitudinal section when viewed from the left of the needle assembly illustrated in FIG. 2;
FIG. 4 is a side view when viewed from the right of the needle assembly illustrated in FIG. 2;
FIG. 5 is an enlarged partial cross-section taken along arrow X-X of FIG. 3;
FIG. 6 is an enlarged schematic front view of the tip of the needle cannula of the needle assembly illustarated in FIG. 2, said tip being at the side covered with the elastomeric resilient cap;
FIG. 7 is a schematic rear view of the needle tip illustrated in FIG. 6;
FIG. 8 is a schematic side view of the needle tip illustrated in FIG. 6;
FIG. 9 is a schematic longitudinal section illustrating a modified embodiment of the invention;
FIG. 10 is a schematic longitudinal section illustrating another embodiment of the invention;
FIG. 11 is a schematic longitudinal section illustrating another modified embodiment of the invention;
FIG. 12 is a schematic longitudinal section illustrating still another modified embodiment of the invention;
FIG. 13 is a view similar to FIG. 5 but illustrating another modified example of the essential portion of the needle assembly illustrated in FIGs.2-5;
FIG. 14 is a view similar to FIG. 5 but illustrating a further modified example of the essential portion of the needle assembly illustrated in FIGs.2-5;
FIG. 15 is a schematic longitudinal section illustrating still another modified embodiment of the invention; and
FIG. 16 is a schematic longitudinal section illustrating a modification of the embodiment of FIG. 15.

### DETAILED DESCRIPTION

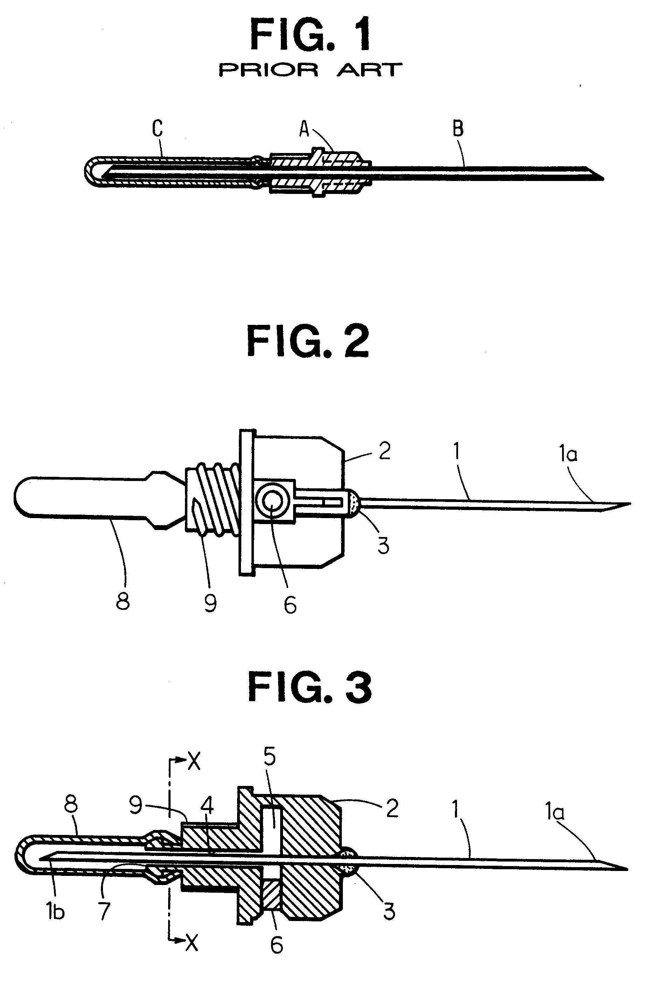

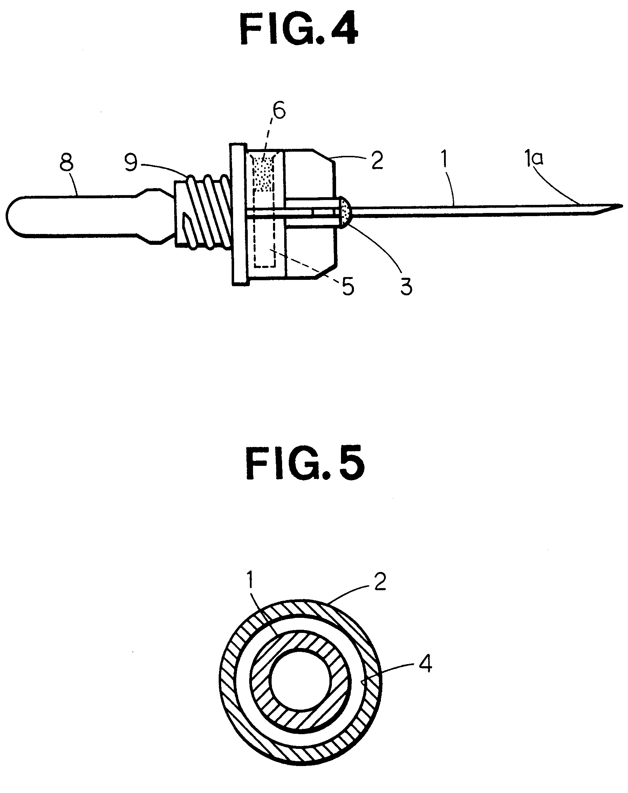

Referring to the drawings, FIGs. 2 to 5 illustrate one embodiment of the present invention. The reference numeral 1 designates a stainless needle cannula, one end 1a of which is formed to be inserted into the vein of a blood examinee, and the other end 1b to be inserted into a vacuum,blood collection container (substantially tube) not shown. A single needle cannula support 2 made of a transparent material is secured liquid-tight, with an adhesive 3, as shown, to be middle portion of the needle cannula 1 at the side of said one end 1a. Further, as shown in FIG. 3, the needle cannula support 2 is internally formed with a longitudinal path 4 opening at the other end 1b side of the needle cannula 1 along the outer circumference of said cannula and forming a guide portion for confirmation blood, and with a radial path 5 having a circular shape in section, said radial path extending radially and outwardly from the inward end of said longitudinal path 4 and communicating with the outside. As illustrated in FIG. 5, the longitudinal path 4 is annularly formed in the circumference of the needle cannula 1. The inner edge of the opening end of said radial path 5 is spreading in tapering shape, the inside of the radial path is anchored with a mass 6 made of paper or synthetic resin, said mass serving as an air-permeable, liquid repellant plug, and while the inside of the longitudinal path 4 is retaining air-permeability the mass 6 as plug prevents blood from leaking to the outside. As the paper mass 6 is used such one that a paper having a suitable thickness is coated with starch component such as corn starch to an extent ranging from several percent to 30 percent, the paper is rolled cylindrically, and then it is dried.

Then, the opening end of the longitudinal path 4 of said needle cannula support 2 is formed with a flange 7 which anchors an elastomeric resilient cap 8 that sealingly covers the other end 1b side of the needle cannula 1 and that is composed of an elastomeric resilient material such as rubber, and the receiving portion of the blood collection container is engaged with a threaded portion 9.

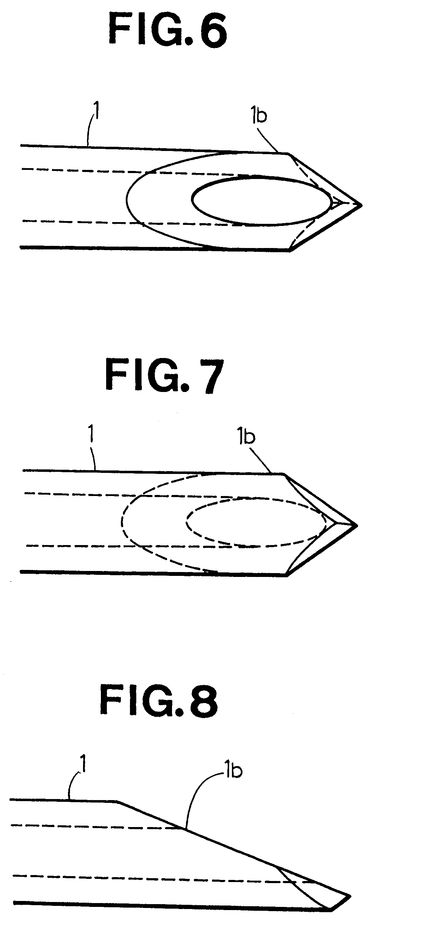

As illustrated in FIGs. 6 to 8, the pointed tip of the other end 1b of the needle cannula 1 is constructed in such a manner that its tip edge of the obliquely cut end face is cut at both its sides from the rear side, it extends in parallel with the axis of the needle cannula, and it is positioned more inwardly than the outer circumference of the needle cannula 1. Therefore, when the elastomeric resilient cap is mounted in the manufacturing procedure there is no longer a fear that the cap 8 might be hooked by the needle tip to cause impairing. On the other hand, the cap 8 is less damaged when the vacuum, blood collection container is mounted or dismantled, and the sealabaility can be sufficiently ensured even if the blood collection containers are repeatedly replaced.

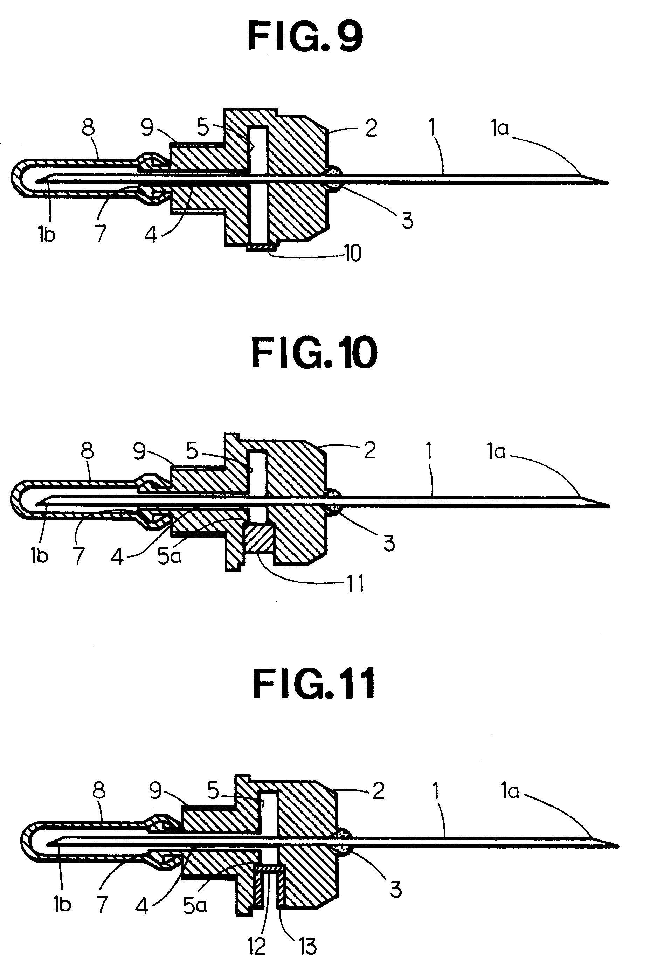

FIG. 9 shows a modified embodiment of the present invention, wherein as an air-permeable, liquid repellant plug which is fitted into the radial path 5 provided in the needle cannula support 2, a paper or synthetic resinous, film-like plug 10 is adhered in place of a paper mass 6 from the outside to the opening of the radial path 5.

FIG. 10 shows another embodiment of the invention, wherein the radial path 5 arranged in the needle cannula 2 has a shoulder 5a near its opening, the side outer than the shoulder is made larger in diameter, and the outer side is fitted with a paper or synthetic resinous mass 11 serving as an air-permeable, blood repellant plug.

FIG. 11 illustrates a modification of the embodiment shown in FIG. 10, in which modification, as the air-permeable, blood repellant plug is fitted, instead of the mass 11, a plug sticked with a paper or synthetic resinous, film-like plug 12 being abutted against the shoulder 5a at the end of a cylindrical holding member 13. In such case, the film-like plug 12 may alternatively be sticked directly to the shoulder 5a in the radial path 5.

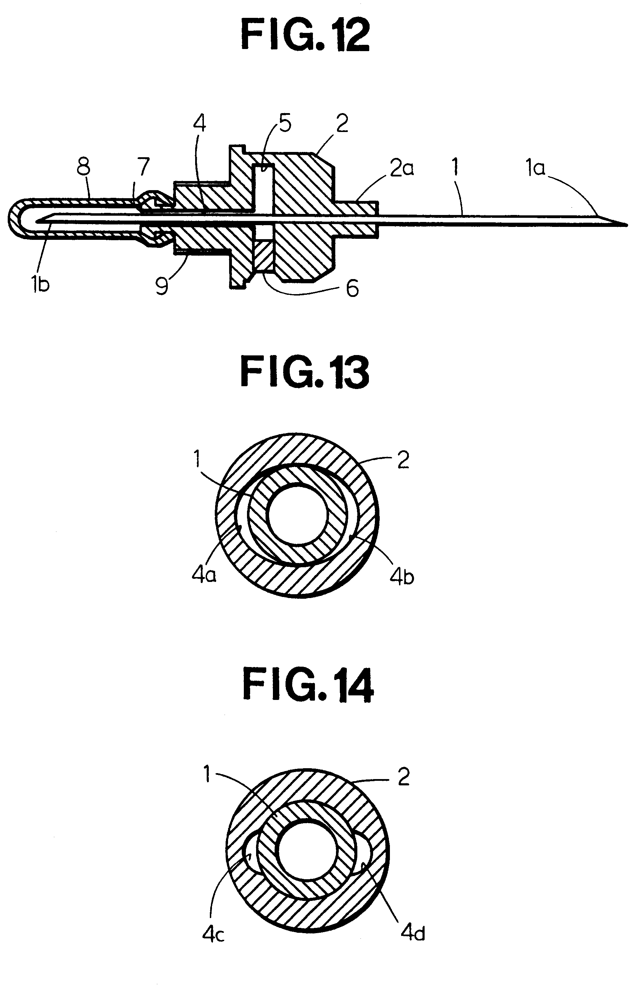

FIG. 12 illustrates still another modified embodiment of the invention, wherein one of the needle cannula support 2 is protruded as shown with the reference numeral 2a, and at that protruded end the needle cannula support 2 is secured under high-frequency melting to the needle cannula 1.

FIGs. 13 and 14 illustrate modified examples of the longitudinal path 4 formed in the needle cannula support 2. In case of FIG. 13 the longitudinal path 4 is of oval shape in section, and it makes longitudinally a line contact at the shorter axis side with the outer periphery of the needle cannula 1 thereby forming at the longer axis side two passages 4a, 4b having a crescent shape in section. On the other hand, in FIG. 14, the needle cannula support 2 makes in the longitudinal direction an arc-like, surface contact with the outer periphery of the needle cannula 1 thereby partitioning the blood introducing pass by two grooves 4c, 4d formed longitudinally in the needle cannula support 2.

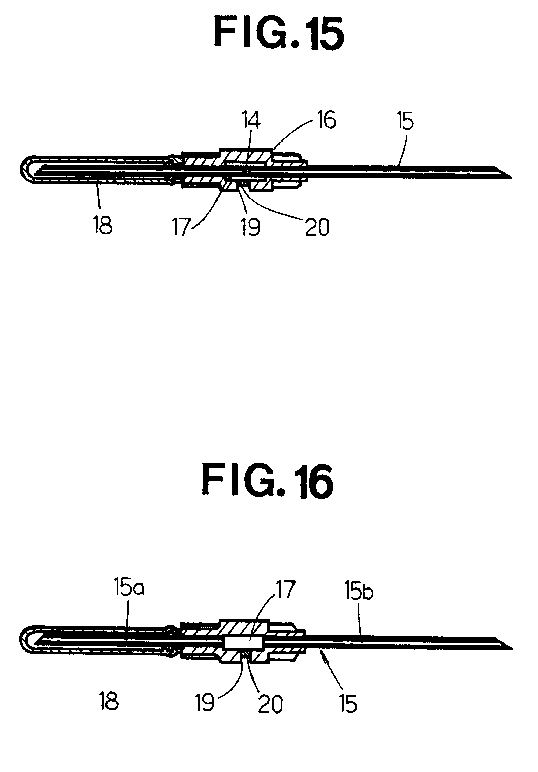

FIG. 15 illustrates still another modified embodiment of the inventionfurther embodiment in which a hole 14 is made at the middle portion of a needle 15, said middle portion is covered with a single translucent or transparent resinous cover or support 16, and a middle hollow portion 17 is provided in said support 16. A clearance is provided between the needle portion covered with a rubber cap 18 and the middle hollow portion 17. The the needle 15 within the translucent or transparent resinous cover 16 is sealingly fixed. On the other hand, the middle hollow portion 17 is provided with an opening 19 while this opening 19 is provided with a water-repellant synthetic resinous body 20 which is used in the form of either solid or membrane depending on use conditions.

FIG. 16 illustrates a modification of the embodiment shown in FIG. 15, in which the needle 15 is separated into two needle portions 15a and 15b.

The resinous body 20 has properties that air permeates therethrough but blood or liquid does not. It is important that blood smoothly flows into the middle hollow portion 17 from the hold 14 and the needle 15 at the rubber cap 18 side when the end tip of the needle 15 has entered the vein of an upper arm of a blood examinee having normal blood pressure, that the blood flowed out never flows to the outside of the resinous body 20, and that even if suction were carried out from the rubber cap 18 side in the vacuum, evacuated blood collection tube or container the outside air is never sucked in.

To discuss the function of the needle assembly illustrated in FIG. 15, it is possible to confirm that if the end tip of the needle 15 correctly enters the vein, blood flows out of the hole 14 thereby coloring in red the interior of the middle hollow portion 17, and that said end tip is in a certain insertion in the vein. This enable even an unskilled user to be able to collect blood easily with the needle assembly of the present invention.

When the end tip of the needle 15 correctly enters the vein of a blood examinee, blood flows into the middle hollow portion 17 of the resinous support 16, so that the correct insertion of the needle can be confirmed with unaided eyes. Then the rubber plug of the vacuum, evacuated blood collection tube is pressed toward the needle side covered with the rubber cap, when the blood easily flows into the tube, whereby it is possible to collect blood with a single operation for a plurality of evacuated blood collection tubes.

In the needle assembly for evacuated blood collection tubes, being thus constructed as illustrated in the drawings, such an air-permeable, blood repellant plug was used that a paper was coated with starch component like corn starch, the paper was rolled cylindrically, and the paper cylinder (being 2.4 mm in diameter and 3.0 mm long) was dried. The air permeability of the paper cylinder was measured with the following result obtained.

In the testing, blood was put into a container (tube) which can be sealed with a rubber plug, and the needle assembly was inserted into the rubber plug while retaining the pressure within the container at certain levels by altering the pressure range from 40.0 g/m² to 7.0 g/m² whereby observation was carried out for each of the pressure levels. In that case, as the pressure gauge was used HANDY MANOMETER PG-100-101G (made by Copal Denshi KK).

| Pressure in container (g/m²) | Flow-in state of blood into needle cannula support 2 |
|---|---|
| 40.0 | Very good |
| 30.0 | Very good |
| 20.0 | Very good |
| 15.0 | Good |
| 10.0 | It took about one second |
| 7.0 | Hardly flowed-in |

By the way, usually human's blood is evacuated through vein, and venous pressure is different from arterial blood generally called blood pressure. The venous pressure varies principally related to the vertical height between the measuring region and the position of the examinee's heart. The following are the data as a result of having measured the venous pressure of 20 persons in their blood evacuating posture.

| | |
|---|---|
| Pressure at the time 0f normal evacuation in a sitting posture: | 65.0 - 73.0 g/m² |
| Pressure at the time of normal evacuation in the supine posture: | 50.0 - 60.0 g/m² |

Alternatively, in the above testing, 150.0 g/m² was the upper limit measured in the resisting pressure under which the air could be discharged and the blood could be stopped from flowing in case the liquid repellant plug absorbed the blood to expand.

It will be understood from this testing result that the needle assembly of the present invention is quite enough to be used if the venous pressure is 15.0 g/m² or higher, that it safely functions up to the pressure double normal venous pressure, and that it therefore can be used in the range of normal venous pressure without any trouble.

In the embodiments illustrated in the drawings there is used, as the air-permeable liquid repellant plug, such one that a paper is coated with starch component such as corn starch and the paper is dried in the form of film or mass, or such one that a synthetic resinous material is impregnated with starch component. However, a plug made of paper itself will be more advantageous from the viewpont of manufacturing cost.

Alternatively, the air-permeable, liquid repellant plug can be provided in double stacking.

Referring to the radial path it is possible that it is provided radially piercing the needle cannula support, and an air-permeable, liquid repellant plug is provided at both the opening of said radial path.

As described above in detail, according to the vacuum, blood collection needle assembly of the present invention, a needle cannula support made of a translucent or transparent material is provided at the middle portion of a single needle cannula, a blood flow confirmation passage is formed within the needle cannula support, said passage communicating with one end of the needle cannula while said end being covered with an elastomeric resilient cap, and one opening end of said passage is provided with an air-permeable, blood repellant plug. At blood evacuation, therefore, it is capable of easily confirming by inserting the needle into the blood examinee's vein whether the needle is in correct insertion or not. Moreover, at the time of collecting blood into a vacuum, blood collection container, the needle cannula itself communicates with only the blood collection container whereas it is shielded from the blood flow confirmation passage within the needle cannula whereby a desired amount of blood can be collected through the needle cannula. Accordingly, not only practical blood collection operation becomes easier but also the frequency of re-insertion of the needle can be reduced so as to lessen the examinee's discomfort.

Further, since the vacuum, needle assembly for blood collection according to the invention employs a needle cannula support comprising a single moulding body and since the air-permeable, liquid repellant plug can be made of paper it is possible to provide the same without substantially increasing the manufacturing cost compared with conventional needle assemblies.

Furthermore, according to the vacuum needle assembly for blood collection of the invention, the end of the needle cannula, which is covered with the elastomeric resilient cap, extends in parallel with the axis of the needle cannula and is pointed in shape while being positioned inner than the outer periphery of the needle cannula, and therefore the cutting opening of said cap is neither broadened nor hooked so that the sealability of the elastomeric resilient cap can be sufficiently retained and the operability of the needle assembly can be improved.

Furthermore, even in the manufacturing procedure, only one constitutional component increases and the structure itself of the needle cannula support is not complicated, so that the needle assembly for evacuated blood collection tubes is structurally simplified compared with those conventionally proposed wheren the flow of blood can be confirmed, and it is possible to plan to largely reduce the manufacturing cost.

## Claims

1. A needle assembly for use with an evacuated blood collection container to draw blood from a blood examinee, said needle assembly comprising: a needle cannula (1), a first end (1a) of which is suitable for insertion into the vein of a blood examinee and a second obliquely cut end (1b) having a tip edge and a rear side of which is suitable for insertion into a vacuum blood collection container which is sealingly attachable to said needle cannula for forming a communication with the interior of the vacuum blood collection container; a needle cannula support (2) mounted at the middle portion of the needle cannula (1), such that said first end (1a) of said needle cannula (1) extends outwardly from and is liquid tightly fixed to a first side of said support and said second end (1b) of said needle cannula (1) extends outwardly from an opposite second side of said support; and an elastomeric resilient cap (8) mounted on the support (2) over said second end (1b) of said needle cannula (1); wherein said support (2) is a single body made of a translucent or transparent material, and wherein said support (2) is provided with a longitudinal passage (4) which is opened toward said opposite second side of the support (2), and extends along the outer periphery of the needle cannula (1) up to an inner end within said support (2), and is further provided with a radial passage (5) extending radially across said longitudinal passage (4) and communicating with the outside, at least one air permeable, liquid repellent plug (6; 10; 11; 12) being anchored in said radial passage (5), characterized in that the tip edge of the obliquely cut end face is cut at both its sides from the rear side so that the second end (1b) of said needle cannula (1) has a sharpened point which lies in a plane including the axis of the needle cannula (1) and is positioned between the axis of the needle cannula and the outer surface of the needle cannula (1).

2. A needle assembly as set forth in claim 1, wherein the side wall of said longitudinal passage (4) is in partial contact with the outer surface of the needle cannula (1).

3. A needle assembly as set forth in claim 1, wherein said longitudinal passage (4) in the needle cannula support (2) has a circular cross section and is formed around the needle cannula (1).

4. A needle assembly as set forth in claim 2, wherein the longitudinal passage (4) in the needle cannula support (2) has an oval shape cross section.

5. A needle assembly as set forth in claim 1 or 2, wherein the longitudinal passage (4) in the needle cannula (1) comprises a plurality of passages (4a, 4b, 4c, 4d) extending lengthwise along the outer periphery of the needle cannula (1).

6. A needle assembly as set forth in one of more of the preceding claims, wherein said air-permeable, liquid repellent plug (8) is made of a synthetic resinous mass.

7. A needle assembly as set forth in one of more of the preceding claims, wherein the inner edge of the end of said radial passage which opens to the outside is tapered outwardly.

8. A needle assembly as set forth in one of more of the preceding claims, wherein the needle cannula support consists of a single molding.

## Patentansprüche

1. Nadelvorrichtung für einen unter Unterdruck stehenden Blutsammelcontainer zum Abziehen von Blut von einem Blutspender, wobei die Nadelanordnung aufweist: Eine Nadelkanüle (1), deren erstes Ende (1a) für die Einführung in die Vene eines Blutspenders geeignet ist, und deren zweites schräg-geschnittenes Ende (1b) mit einer Spitzenkante und einer Rückseite, die für die Einführung in einen unter Unterdruck stehenden Blutsammelcontainer geeignet ist, der dichtend mit der Nadelkanüle verbindbar ist, um eine Verbindung mit dem Inneren des unter Unterdruck stehenden Blutsammelcontainers zu bilden, eine Nadelkanülen-Trägervorrichtung (2), die beim Mittelabschnitt der Nadelkanüle (1) angebracht ist, so daß das erste Ende (1a) der Nadelkanüle (1) sich nach außen erstreckt und flüssigkeitsdicht mit einer ersten Seite der Trägervorrichtung verbunden ist, und so daß das zweite Ende (1b) der Nadelkanüle (1) sich nach außen einer entgegengesetzten zweiten Seite der Trägervorrichtung erstreckt, und eine elastische, federnde Kappe (8), die auf der Trägervorrichtung (2) über dem zweiten Ende (1b) der Nadelkanüle (1) angebracht ist, wobei die Trägervorrichtung (2) ein einzelner Körper aus einem durchsichtigen oder transparenten Material ist, und wobei die Trägervorrichtung (2) mit einem longitudinalen Durchgang (4) ausgestattet ist, der hin zu der entgegengesetzen zweiten Seite der Trägervorrichtung (2) geöffnet ist, und der sich entlang dem äußeren Rand der Nadelkanüle (1) hin zu einem inneren Ende innerhalb der Trägervorrichtung (2) erstreckt, und daß sie desweiteren einen radialen Durchgang (5) aufweist, der sich radial quer zu dem longitudinalen Durchgang (4), erstreckt und mit der Außenseite in Verbindung steht, wobei zumindest ein luftdurchlässiger, flüßigkeitabweisender Pfropfen (6;10;11;12) in dem radialen Durchgang (5) verankert ist, **dadurch gekennzeichnet,** daß die Spitzenkante der geneigt geschnittenen Endfläche an seinen beiden Seiten von der Rückseite her geschnitten ist, so daß das zweite Ende (1b) der Nadelkanüle (1) einen spitzzulaufenden Punkt hat, der in einer Ebene liegt, welche die Achse der Nadelkanüle(1) einschließt, und der zwischen der Achse der Nadelkanüle(1) und der äußeren Oberfläche (1) der Nadelkanüle (1) angeordnet ist.

2. Nadelanordnung nach Anspruch 1, bei der die Seitenwand des longitudinalen Durchgangs (4) teilweise mit der äußeren Oberfläche der Nadelkanüle (1) in Kontakt steht.

3. Nadelanordnung nach Anspruch 1, bei der der longitudinale Durchgang (4) in der Nadelkanülen-Trägervorrichtung (2) einen kreisförmigen Querschnitt hat und um die Nadelkanüle (1) herum ausgebildet ist.

4. Nadelanordung nach Anspruch 2, bei der der longitudinale Durchgang (4) in der Nadelkanülen-Trägervorrichtung (2) einen ovalen Querschnitt hat.

5. Nadelanordnung nach Anspruch 1 oder 2, bei der der longitudinale Durchgang (4) in der Nadelkanüle (1) eine Vielzahl von Durchgängen (4a,4b,4c,4d) aufweist, die sich entlang des äußeren Randes der Nadelkanüle (1) erstrecken.

6. Nadelanordnung nach einem oder mehreren der vorangehenden Ansprüche, bei der der luftdurchlässige, flüssigkeitabweisende Pfropfen (6) aus einer synthetischen Harzmasse gebildet ist.

7. Nadelanordnung nach einem oder mehreren der vorangehenden Ansprüche, bei der die innere Kante des Endes des radialen Durchgangs, das sich nach außen öffnet, nach außen kegelförmig ist.

8. Nadelanordnung nach einem oder mehreren der vorangehenden Ansprüche, bei der die Nadelkanülen-Trägervorrichtung aus einem Guß gebildet ist.

## Revendications

1. Un ensemble à aiguille destiné à être utilisé avec un récipient de recueil de sang à dépression pour extraire du sang d'un patient, ledit ensemble à aiguille comprenant une canule d'aiguille (1) dont une première extrémité (1a) est appropriée pour l'insertion dans la veine d'un patient et dont une deuxième extrémité coupée de façon oblique (1b) ayant un bord d'extrémité et des côtés arrière est appropriée pour l'insertion dans un récipient de recueil de sang sous vide qui est apte à être attaché de façon étanche à ladite canule d'aiguille pour former une communication avec l'intérieur du récipient de recueil de sang sous vide, un support de canule d'aiguille (2) monté à la partie médiane de la canule d'aiguille (1) si bien que ladite première extrémité (1a) de ladite canule d'aiguille (1) s'étend vers l'extérieur et est fixée de façon étanche au liquide à un premier côté dudit support et ladite deuxième extrémité (1b) de ladite canule d'aiguille (1) s'étend vers l'extérieur depuis un deuxième côté opposé dudit support et un capuchon résilient élastomère (8) monté sur le support (2) sur ladite deuxième extrémité (1b) de ladite canule d'aiguille (1), dans lequel ledit support (2) est un seul corps réalisé en matériau translucide ou transparent et dans lequel ledit support (2) est muni d'un passage longitudinal (4) qui est ouvert vers ledit deuxième côté opposé du support (2) et s'étend le long de la périphérie extérieure de la canule d'aiguille (1) jusqu'à une extrémité intérieure dans ledit support (2) et est muni, en outre, d'un passage radial (5) s'étendant radialement au niveau dudit passage longitudinal (4) et communiquant avec l'extérieur, au moins un bouchon repoussant le liquide, perméable à l'air (6, 10, 11, 12) étant ancré dans ledit passage radial (5), caractérisé en ce que le bord d'extrémité de la face d'extrémité coupée de façon oblique est coupé à ses deux côtés depuis le côté arrière si bien que la deuxième extrémité (1b) de ladite canule d'aiguille (1) présente une partie aiguisée qui s'étend dans un plan comprenant l'axe de la canule d'aiguille (1) et est positionnée entre l'axe de la canule d'aiguille et la surface extérieure de la canule d'aiguille (1).

2. Un ensemble d'aiguille selon la revendication 1, dans lequel la paroi latérale dudit passage longitudinal (4) est en contact partiel avec la surface extérieure de la canule d'aiguille (1).

3. Un ensemble d'aiguille selon la revendication 1, dans lequel ledit passage longitudinal (4) dans le support de canule d'aiguille (2) présente une section transversale circulaire et est formé autour de la canule d'aiguille (1).

4. Un ensemble d'aiguille selon la revendication 2, dans lequel le passage longitudinal (4) dans le support de canule d'aiguille (2) présente une section transversale de forme ovale.

5. Un ensemble d'aiguille selon la revendication 1 ou 2, dans lequel le passage longitudinal (4) dans la canule d'aiguille (1) comprend une pluralité de passages (4a, 4b, 4c, 4d) s'étendant dans le sens longitudinal le long de la périphérie extérieure de la canule d'aiguille (1).

6. Un ensemble d'aiguille selon l'une quelconque des revendications précédentes, dans lequel ledit bouchon repoussant le liquide perméable à l'air (8) est réalisé en une masse résineuse synthétique.

7. Un ensemble d'aiguille selon l'une quelconque des revendications précédentes, dans lequel le bord intérieur de l'extrémité dudit passage radial qui débouche à l'extérieur est effilée vers l'extérieur.

8. Un ensemble d'aiguille selon l'une quelconque des revendications précédentes, dans lequel le support de canule d'aiguille se compose d'un seul moulage.
